# EUROPEAN PATENT SPECIFICATION

(11) **EP 0 517 160 B1**
(45) Date of publication and mention of the grant of the patent: **13.03.1996**
(21) Application number: 92109289.6
(22) Date of filing: 02.06.1992
(51) Int. Cl.: A61K 9/06

(54) **Extended-release ophthalmic preparations**
Augenpräparate mit verlängerter Freigabe
Préparations ophtalmiques à libération prolongée

(30) Priority: 07.06.1991 IT MI911580
(43) Date of publication of application: 09.12.1992
(73) Proprietor: INVERNI DELLA BEFFA S.P.A., I-20141 Milano (IT)
(72) Inventor: Seghizzi, Roberto, I-20141 Milano (IT); Malandrino, Salvatore, I-20141 Milano (IT); Vitali, Romano, I-20141 Milano (IT); Pifferi, Giorgio, I-20141 Milano (IT)
(74) Representative: Minoja, Fabrizio

(56) References cited:
- EP-A- 0 253 472
- EP-A- 0 317 405
- FR-A- 2 353 295
- GB-A- 2 203 039
- US-A- 4 188 373
- US-A- 4 474 751
- US-A- 4 476 140
- CHEMICAL ABSTRACTS, vol. 110, no. 22, 29 May 1989, Columbus, Ohio, US; abstract no. 199306X, C. VERTUSCHI ET AL: 'application of third derivative uv spectrophotometry to the simultaneous determination of n-methyl-5,6- benzoquinoline methyl sulfate and thonzylamine hydrochloride, in multicomponent collyrium' page 430 ;column 1 ;& spectrosc. lett. 1989, 22(1), 41-9

## Description

The present invention relates to extended-release ophthalmic preparations containing forskoline or other active principles employed in ophthalmology.

Ophthalmic formulations in general contain the active principle or principles in the form of a solution or suspension. The latter type of formulation exhibits, as compared with solutions, various disadvantages deriving, for example, from the difficulties of resuspendibility, with consequent lack of constancy of dosage and reduced bioavailability. Moreover, the formulations in suspension may give rise to problems regarding an increase in the particle size and variations of crystalline habit, with possible consequent eye irritations and compromising of the stability of the preparation.

The administration of an ophthalmic pharmaceutical product in the form of a conventional aqueous solution, where this is permitted by the physico-chemical characteristics of the active principle, may eliminate such disadvantages, but, in turn, exhibits other limitations due to the protective physiological mechanisms of the eye, such as reflex lachrymation, winking and drainage, which rapidly reduce the concentration and the dwell time of the pharmaceutical product which has been instilled. Accordingly, there is a medical need for ophthalmic formulations which have a long period of action and contain active principles of varying type.

The subject of the present invention are pharmaceutical formulations for ophthalmic use, in which the active principle is contained in high-viscosity solutions. Such formulations, which may assume the appearance of viscous liquids or of a gel, are stable under normal conditions of use and exercise a prolonged action.

The proposed formulations permit the active principles to be maintained in solution in the conjunctival sac in such a manner as to reduce the dosage and frequency of the administrations, with the advantage of thus improving the patient compliance and the local tolerability.

U.S. Patents 4,100,271 and 4,188,373 describe, for topical application to the mucosae, preparations based on "poloxamers", which are liquid at room temperature and gels at body temperature.

GB-A-2 203 039 describes stable ophthalmic preparations containing acetazolamide, wherein the stability is obtained by controlling pH at values lower than 5.

An illustrative example of the present invention is concerning the ophthalmic formulation of forskoline.

Forskoline, 7β-acetoxy-8,13-epoxy-1α,6β,9α-trihydroxy-labd-14-en-11-one, is a diterpene of the labdane family, which is obtained by extraction from Coleus forskolii. The ocular hypotensive action of forskoline administered topically has been fully demonstrated in the rabbit, in the monkey and also in man, in healthy subjects and in subjects with glaucoma (The Lancet, i, 958, 1983; Clin. Ocul., 9, 173, 1988). The ocular hypotensive action is attributed to a reduction in the production of aqueous humour induced by stimulating the adenylcyclase system in the clear cells of the ciliary epithelium, with aspiration of endocular liquid from the posterior chamber of the eye within the vessels of the ciliary body (Am. J. Ophtalm. 100, 194, 1985).

Forskoline is a solid which is pratically insoluble in water and has up to now been administered to the eye in the form of an aqueous suspension (Lancet, i, 958, 1983; Clin. Ocul., 9, 173, 1988).

Recently, techniques of solubilisation of forskoline in an aqueous medium (J. Ocul. Pharmacol., 5, 111, 1989) have been described, using "poloxamers" and "cyclodextrins" as solubilisers. In particular the poloxamer 407, a polyoxyethylene-polyoxypropylene copolymer in which the number of polyoxyethylene units is approximately 70% of the total number of monomer units and which has an average molecular weight of approximately 12,500, is endowed with high solubility in water and exhibits, at proper concentrations, thermoreversible gelling. The amount of forskoline solubilised in water is directly proportional to the concentration of poloxamer 407. At the maximum tested concentration of polymer (25% w/v), a maximum of 0.12% of forskoline dissolves at 5°C. However, this formulation is not stable, as the active principle precipitates under certain storage conditions, for example in the case of thermal stress. Storage tests under stress conditions (S.S. Davis in: Stability Testing of Drug Products, W. Grimm and K. Tomae, Eds. Wissenschaftliche Verlagsgesellschaft GmbH Stuttgart 1987) are recommended in order to demonstrate the potential instability of the pharmaceutical preparations, which instability may become evident in the course of storage and shipping. Moreover, the "Draft Guideline for Stability Studies for Human Drugs and Biologics" of the Food and Drug Administration recommend that the pharmaceutical preparations should be subjected to temperature variation cycles, so as to simulate the fluctuations which may take place while the drug product is in distribution channels.

It has surprisingly been found that the addition to aqueous solutions of forskoline or other active principles in poloxamers of from 5 to 6% (w/v) of non-ionic surfactants which are physiologically compatible for ophthalmic use prevents the reprecipitation of the active principle, even under the aforementioned stress conditions. Among the non-ionic surfactants which can be used, preference is given in particular to the esters of polyoxyethylene sorbitan with fatty acids (preferably polysorbate 20 and polysorbate 80) and polyethoxylated vegetable oils (preferably polyethoxylated hydrogenated castor oil with approximately 40 moles of ethylene oxide). The surfactants are present in the compositions forming the subject of the invention in amounts of not less than 5% (w/v).

In fact, lower concentrations prevent the precipitation of the active principle at low temperatures only for limited periods and in any case for periods which are too short to permit the marketing thereof.

The poloxamers are present in the formulations in percentages within the range of 15 to 25% w/v, while the active principle is present in percentages within the range of 0.01 to 0.1% w/v. A matter which is surprising and which constitutes one of the principal advantages of the invention, is the fact that, with the compositions of the invention it becomes possible to obtain a therapeutic effect which is more prolonged in terms of time and more marked as compared with that obtained with known compositions containing doses of forskoline or other active principles which are 10 times higher.

The presence of the surfactant moreover ensures an improved miscibility of the preparation with the lachrymal fluids. The formulations forming the subject of the present invention also contain suitable soluble and physiologically compatible salts, which make the formulations isotonic with the lachrymal fluids, regulate the pH thereof within physiological values and protect the preparations from microbial contaminations.

Besides forskoline, the formulations of the present invention may include other active principles, such as:
antiglaucoma agents (e.g. pilocarpine, thymolol);
vasoconstrictors (e.g. naphazoline, tetrazoline);
anti-inflammatory steroids (e.g. hydrocortisone, prednisolone, desamethasone, betamethasone and their esters);
anti-inflammatory non-steroids (e.g. flurbiprofen, diclofenac, ketoprofene, bendazac);
antiallergic agents (e.g. chromoglycate, pheniramine, thonzylamine);
antibiotics (e.g. neomycin, chloroamphenicol, oxytetracycline);
local anaesthetics (e.g. tetracaine).

The formulations of the present invention may further contain associations of two or more aforementioned active principles.

The examples which are set forth hereinbelow illustrate the invention in greater detail.

### EXAMPLE 1

| | |
|---|---|
| Forskoline | 0.1 g |
| Poloxamer 407 | 23 g |
| Polysorbate 20 | 6 g |
| Monobasic sodium phosphate monohydrate | 0.017 g |
| Dibasic sodium phosphate dodecahydrate | 0.528 g |
| Sodium chloride | 0.516 g |
| Benzalkonium chloride | 0.020 g |
| Disodium edetate | 0.1 g |
| Sterile purified water to | 100 g |

The preparation of this formulation involves heating the mixture of forskoline and poloxamer 407 to 60-70°C with continuous stirring until the active principle is completely dissolved. Separately, the remaining excipients are dissolved in water at 70°C and this solution is added to the previously molten mass with stirring.

Cooling takes place to 5°C until a transparent homogeneous liquid is obtained, which has a sol-gel transition temperature not exceeding 16-18°C.

The same procedure has also been used for the preparations described in Examples 2-5.

### EXAMPLE 2

| | |
|---|---|
| Forskoline | 0.1 g |
| Poloxamer 407 | 22 g |
| 40-Polyethoxylated hydrogenated castor oil | 6 g |
| Monobasic sodium phosphate monohydrate | 0.017 g |
| Dibasic sodium phosphate dodecahydrate | 0.528 g |
| Sodium chloride | 0.516 g |
| Benzalkonium chloride | 0.020 g |
| Disodium edetate | 0.1 g |
| Sterile purified water to | 100 g |

The preparation maintains the same physical characteristics as Example 1.

### EXAMPLE 3

| | |
|---|---|
| Forskoline | 0.05 g |
| Poloxamer 407 | 22 g |
| Polysorbate 20 | 6 g |
| Monobasic sodium phosphate monohydrate | 0.017 g |
| Dibasic sodium phosphate dodecahydrate | 0.528 g |
| Dextrose | 2.90 g |
| Benzalkonium chloride | 0.020 g |
| Disodium edetate | 0.1 g |
| Sterile purified water to | 100 g |

The preparation exhibits the same physical characteristics as Examples 1 and 2.

### EXAMPLE 4

| | |
|---|---|
| Forskoline | 0.05 g |
| Poloxamer 407 | 17.5 g |
| Polysorbate 20 | 6 g |
| Monobasic sodium phosphate monohydrate | 0.017 g |
| Dibasic sodium phosphate dodecahydrate | 0.528 g |
| Sodium chloride | 0.552 g |
| Benzalkonium chloride | 0.020 g |
| Disodium edetate | 0.1 g |
| Sterile purified water to | 100 g |

The preparation is in the form of a viscous liquid at room temperature and in the form of a gel at body temperature.

### EXAMPLE 5

| | |
|---|---|
| Forskoline | 0.05 g |
| Poloxamer 407 | 17 g |
| Polysorbate 20 | 6 g |
| Polyvinylpyrrolidone | 1 g |
| Monobasic sodium phosphate monohydrate | 0.017 g |
| Dibasic sodium phosphate dodecahydrate | 0.528 g |
| Sodium chloride | 0.552 g |
| Benzalkonium chloride | 0.020 g |
| Disodium edetate | 0.1 g |
| Sterile purified water to | 100 g |

This preparation remains a viscous liquid, even at body temperature.

### EXAMPLE 6

| | |
|---|---|
| Pilocarpine hydrochloride | 4 g |
| Poloxamer 407 | 18 g |
| Polysorbate 20 | 6 g |
| Sodium chloride | 0.6 g |
| Benzalkonium chloride | 0.02 g |
| Disodium edetate | 0.1 g |
| Sterile purified water | q.s. to 100 g |

The preparation of this formulation involves heating the mixture of pilocarpine, poloxamer 407 and polysorbate 20, to 70°C with continuous stirring. Separately, the remaining excipients in water at 70°C and this solution is added to the previously molten mass with stirring.

Cooling takes place to 5°C until a transparent homogeneous liquid is obtained.

The formulation appears as a viscous liquid at room temperature and as a gel at body temperature.

### EXAMPLE 7

| | |
|---|---|
| Pilocarpine hydrochloride | 4 g |
| Poloxamer 407 | 23 g |
| Polysorbate 20 | 6 g |
| Sodium chloride | 0.6 g |
| Benzalkonium chloride | 0.02 g |
| Disodium edetate | 0.1 g |
| Sterile purified water | q.s. to 100 g |

The formulation is prepared according to the procedure described in Example 6.

The obtained formulation has a sol-gel transition temperature no higher than 16-18°C.

The stability of the formulations of Examples 1 and 2 and of the reference gel (prepared according to J. Ocul. Pharmacol., 5, 111, 1989) was checked in two tests:
- Test A involves the storage of the preparations at a temperature within the range of 3-6°C;
- Test B involves the preparations being subjected to cooling/heating cycles of 24 hours at 3-6°C and 24 hours at room temperature (cycling test).

Table 1 shows the data which relate to the appearance of the preparations at room temperature and at 37°C, before and after the storage conditions A and B.

The activity with respect to the intraocular pressure (IOP) of the formulations forming the subject of the present invention was tested on the eye of rabbits, at normal ocular pressure, after topical application.

The rabbits, placed in a restain cage throughout the test, were subjected to the measurement of the IOP, by means of a Schiötz tonometer, immediately before and 0.5, 1, 2, 4, 8 and 10 hours after the application, to the right eye, of 100 microlitres of the formulation under examination. By way of reference, use was made of 0.1 and 1% forskoline suspensions in methyl cellulose and a 0.12% gel prepared according to J. Ocul. Pharmacol., 5, 111, 1989.

The results set forth in Table 2 indicate that the 0.1% suspension reduces the IOP by approximately 3 mmHg with effect from 30 min after administration, for a maximum period of 1-2 hours. The administration of an equivalent dose of forskoline, formulated according to Example 1, on the contrary, induces a reduction of the IOP which lasts for at least 8 hours. The action peak is within the range of 1 to 2 hours, with a reduction in the IOP of approximately 7 mmHg. The formulation of Example 1 is shown to possess a greater activity and especially a longer period of action, even compared with the 1% suspension and the 0.12% reference gel.

The activity with respect to the IOP was also assessed in the eye of a rabbit which had been made hypertensive by means of injection, into the posterior chamber of the right eye, of 0.5 ml of a solution containing 300 units/ml of α-chymotrypsin (Am. J. Ophthalm., 77, 378, 1974). During the following 15 days, examination of the treated eye and measurement of the IOP was undertaken daily. Those animals which exhibited anomalies of the ocular tissues or excessively low pressure values were excluded from the experiment. On the 16th day, the rabbits were subjected to measurement of the IOP, immediately before and 0.5, 1, 2, 3, 4, 5, 6, 7 and 24 hours after the application, to the treated eye, of 100 microlitres of the formulations under examination.

The results set forth in Table 3 indicate that the 1% forskoline suspension shows a reduction of 5 mmHg half an hour after administration, with an activity which is exhausted within two hours.

The formulations of Examples 4 and 5, containing 0.05% forskoline (concentrations 20 times lower than that of the suspension), have a significant hypotensive ocular activity characterised by a rapid onset and long duration. In fact, the activity peak, approximately -9 mmHg, appears after 1 h and a significant activity is still present 8 hours after administration.

The results of Table 4 show similar properties for the formulations of examples 6 and 7 containing 4% of pilocarpine hydrochloride.

The ocular tolerability of the formulations 1, 4 and 5 was checked in comparison with the corresponding placebos and with the 0.1% suspension of forskoline in Methocel^{R}. One hundred microlitres of the preparations were inserted into the conjunctival sac of the right eye of 6 rabbits. The left eye, which was not treated, was used as a control. After application, the two eyelids were held closed for a few seconds, so as to prevent escape of the product. The eyes were examined after 5 minutes, 1, 4, 6 and 24 hours. The severity of the lesions affecting the conjunctiva (reddening, swelling and lachrymation) was assessed by means of scores according to the Draize scale (J. Pharmacol. Exp. Ther., 81, 377, 1944).

The results set forth in Table 5 indicate that the tested formulations prove to be well tolerated. The 0.1% forskoline suspension exhibits a slight irritant effect, even just five minutes after instillation. These results agree with those described in the literature (Klin. Mol. Augenheilk., 185, 522, 1984) and are to be attributed to the vasodilating properties of the active principle. The irritant effect is of short duration and is linked to the capacity of the eye to "wash away" the instilled suspension rapidly.

Even the formulations forming the subject of the invention induced a slight irritation which appears over longer periods of time and lasts for 6 hours, decreasing with time. This phenomenon confirms the capacity of the formulations forming the subject of the invention to release the active principle in a controlled manner over a period of time. By way of further confirmation that the slight irritation is to be attributed to forskoline, the placebo formulations proved to be entirely free of irritant effect.

The formulations of Examples 1-5 permit forskoline to be kept in poloxamer 407 solution, even under conditions of thermal stress, by means of the addition of suitable amounts of non-ionic surfactants.

The proposed formulations, which are in the form of a gel or viscous liquids, are thus stable both at low temperatures and under conditions of thermal fluctuation while the drug product is in distribution channels. These innovative formulations guarantee the stability of the ophthalmic preparations during their shelf-life, avoiding any requirement for special storage precautions, which would be difficult to observe in the shipping and warehousing phases. Moreover, by modifying the concentration of the poloxamer, it is possible to modulate the viscosity of the preparation in the form of a viscous collyrium or in the form of a gel. In particular, the amount of poloxamer 407 employed in Examples 1, 2 and 3 was selected in relation to the dose of active principle to be solubilised and to the requirement to obtain a gel having optimal characteristics for ocular application.

The optimal characteristics of viscosity of the proposed formulations are reflected in an extension of the contact time of the preparations with the corneal epithelium, together with the advantage of drastically reducing (10-20 times) the active concentration of forskoline and the frequency of administration. This results in an improvement in the patient compliance and local tolerability of the preparation.

The novel ophthalmic formulations maintain the active principles in a stable solution, reduce the dosages thereof, and increase the bioavailability and the ocular tolerability thereof, proving to be particularly efficacious in extending the desired pharmacological effect.

These characteristics exhibit significant advantages in the treatment of the most widely varying ocular disorders.

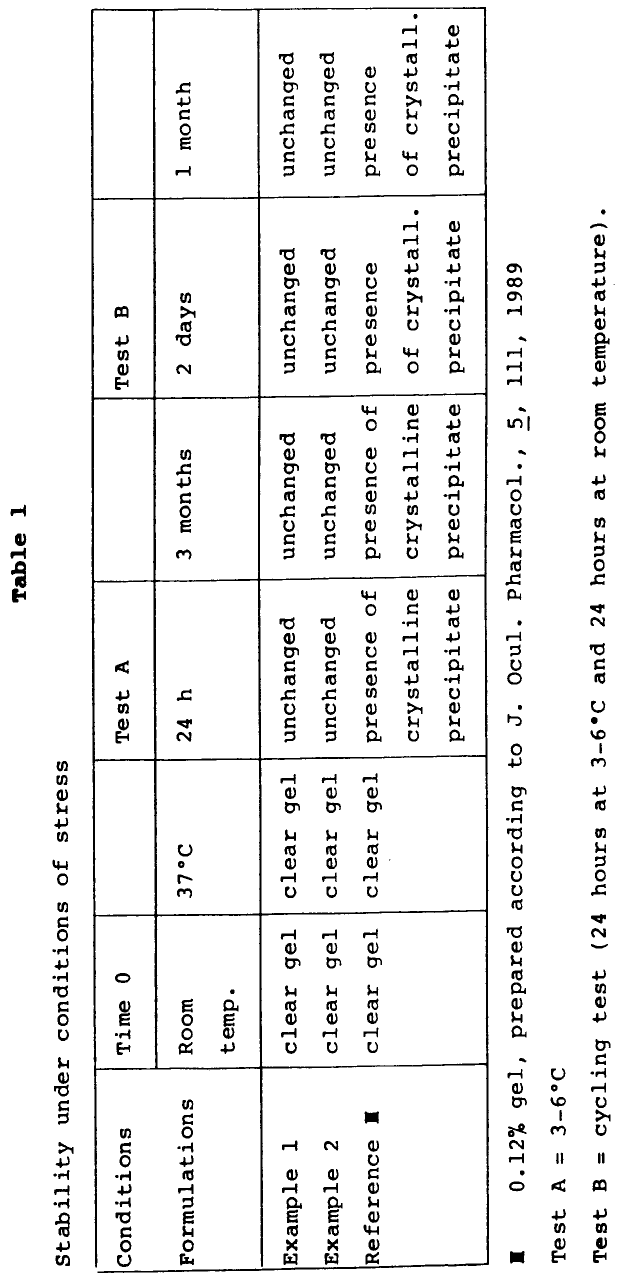

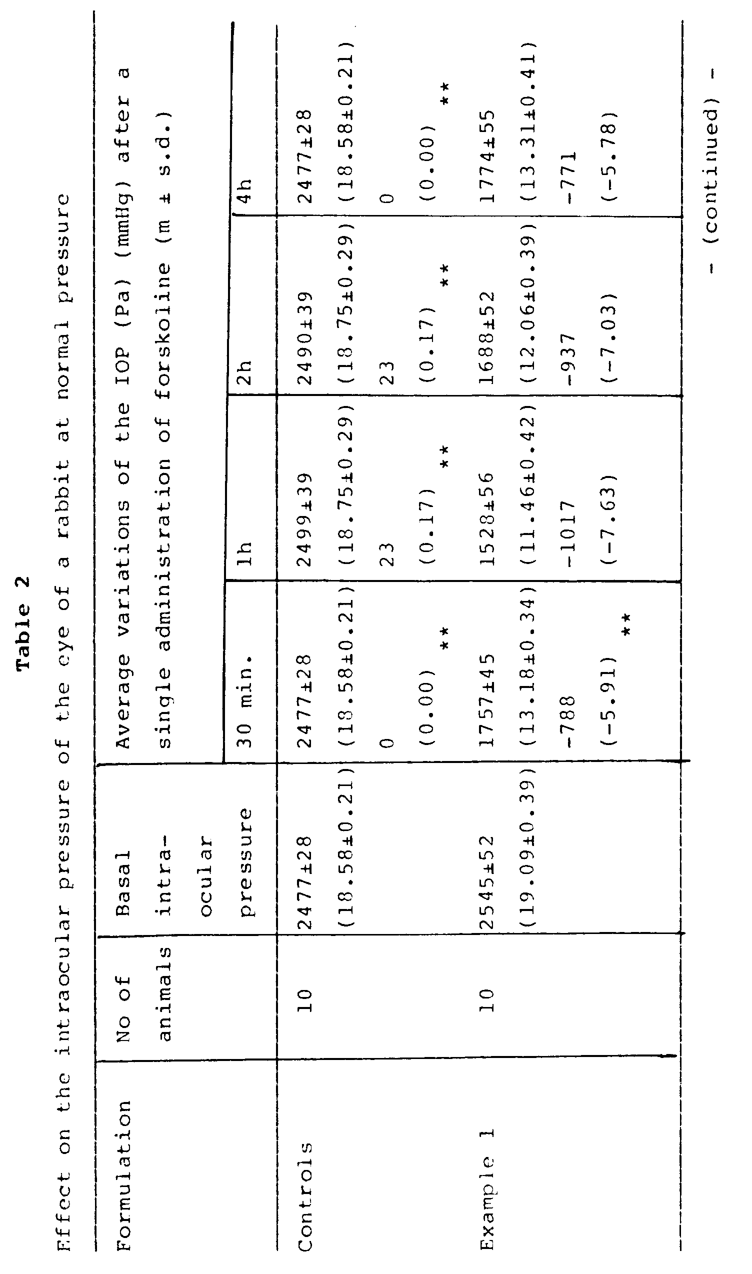

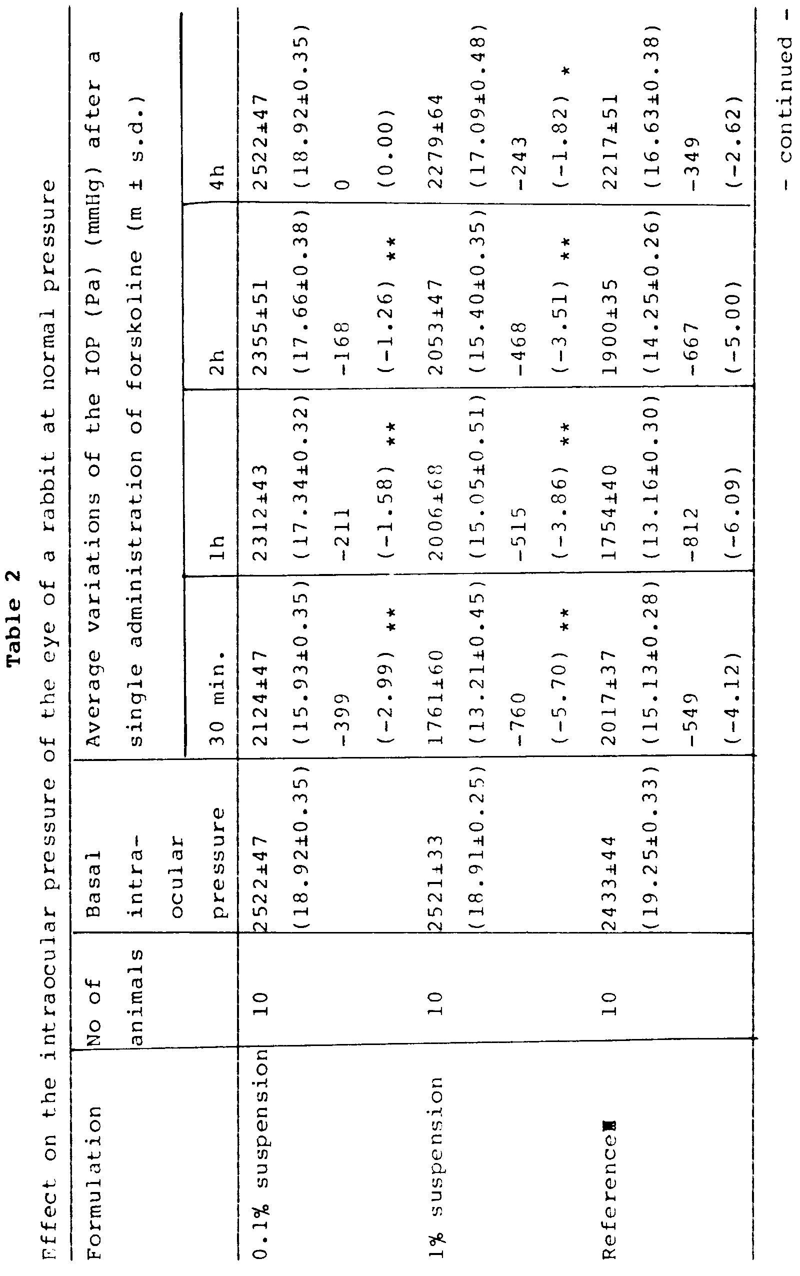

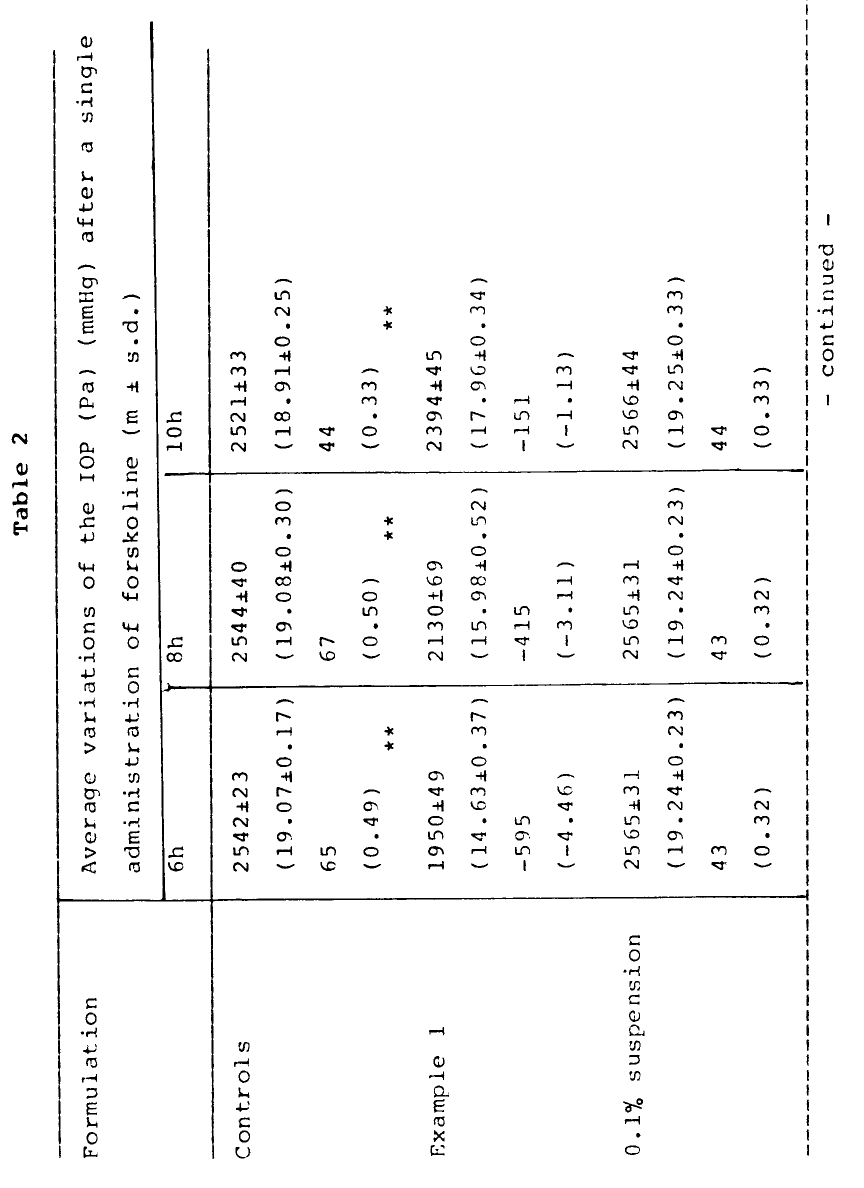

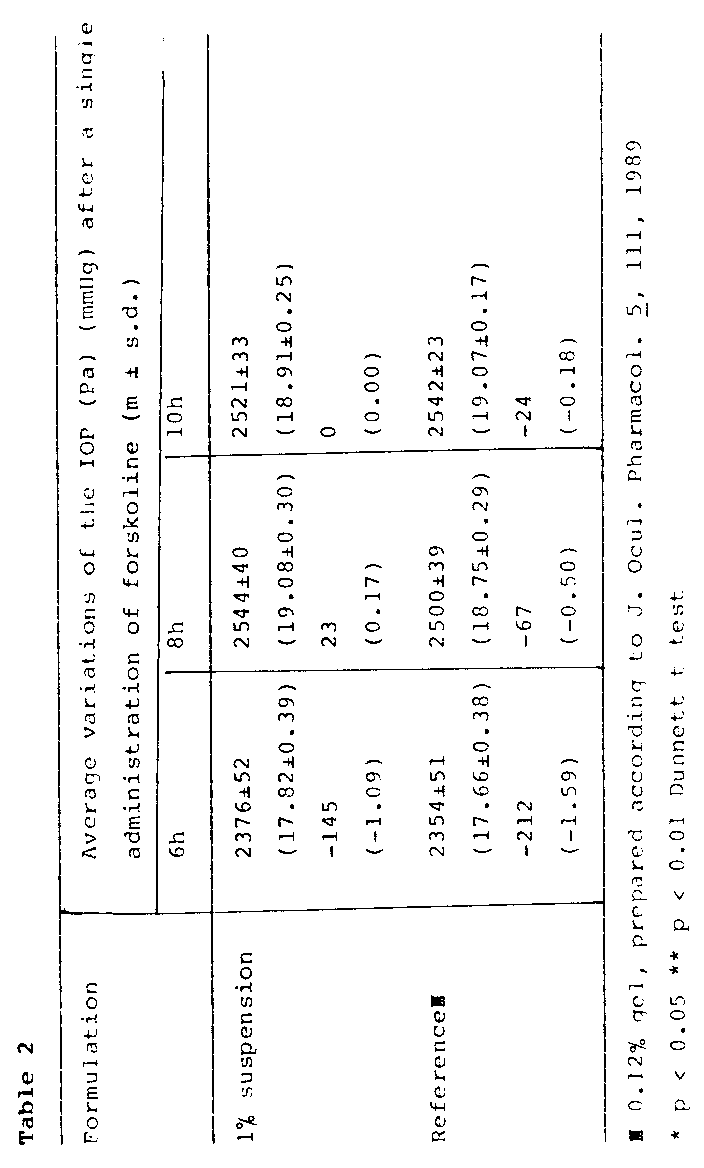

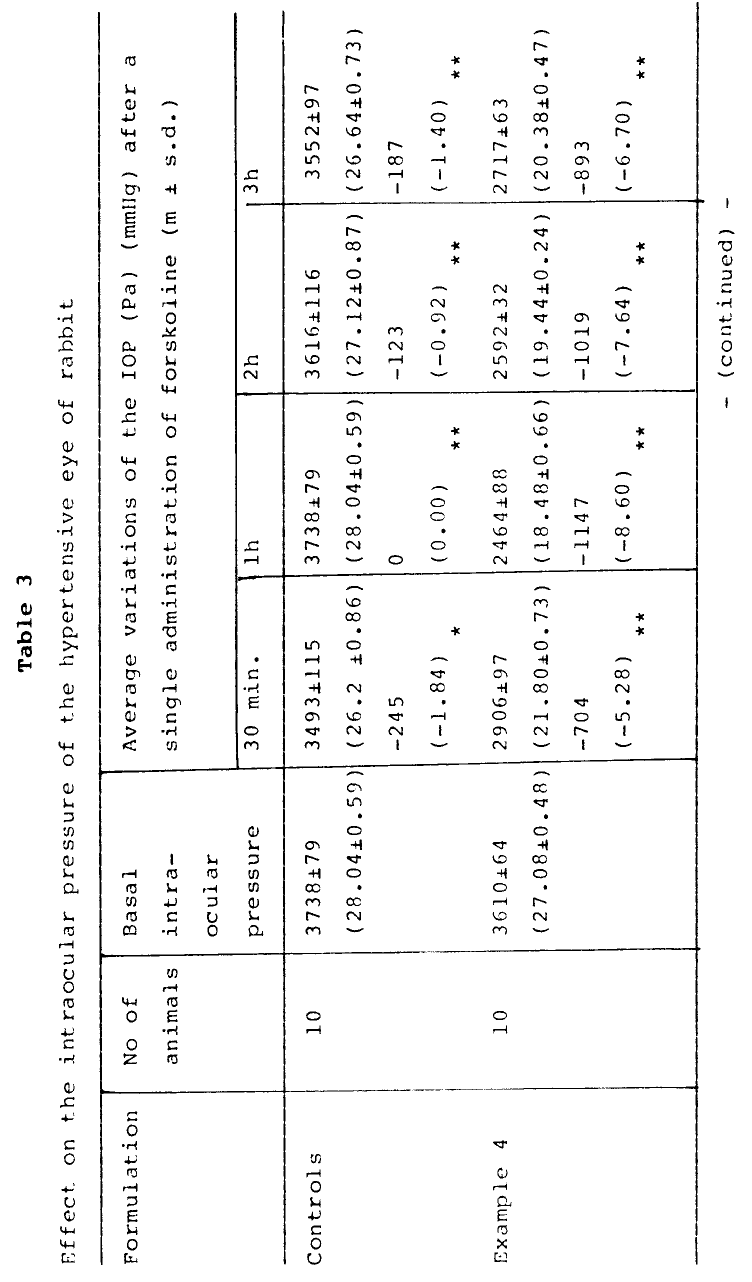

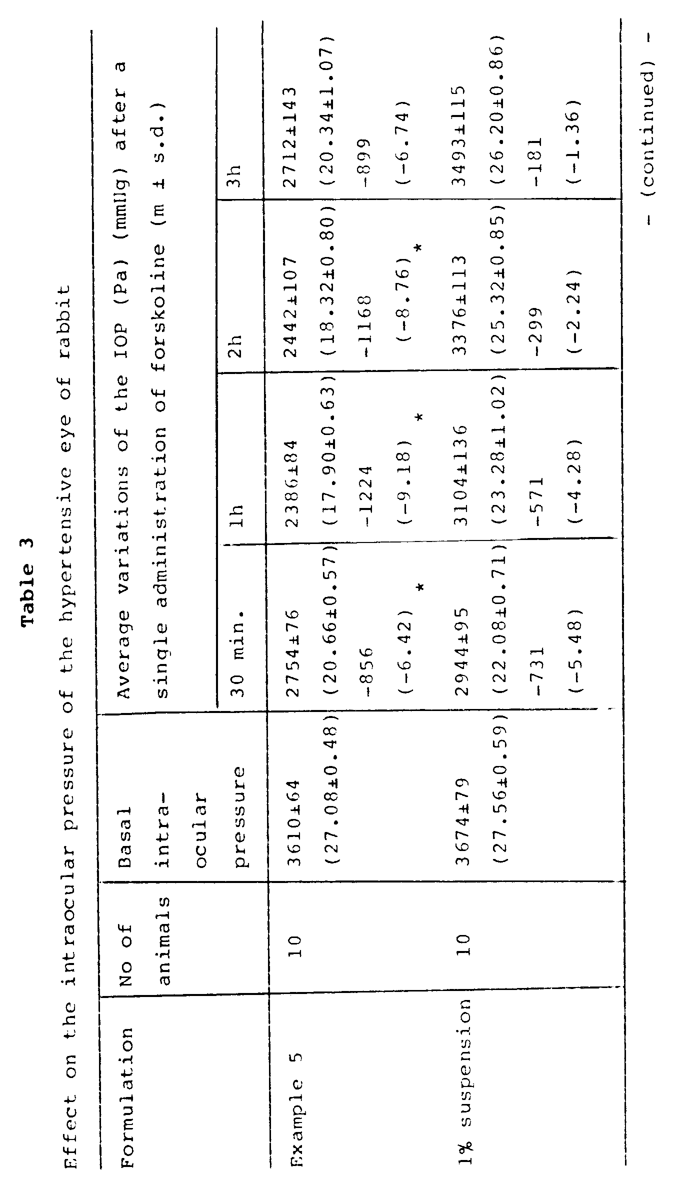

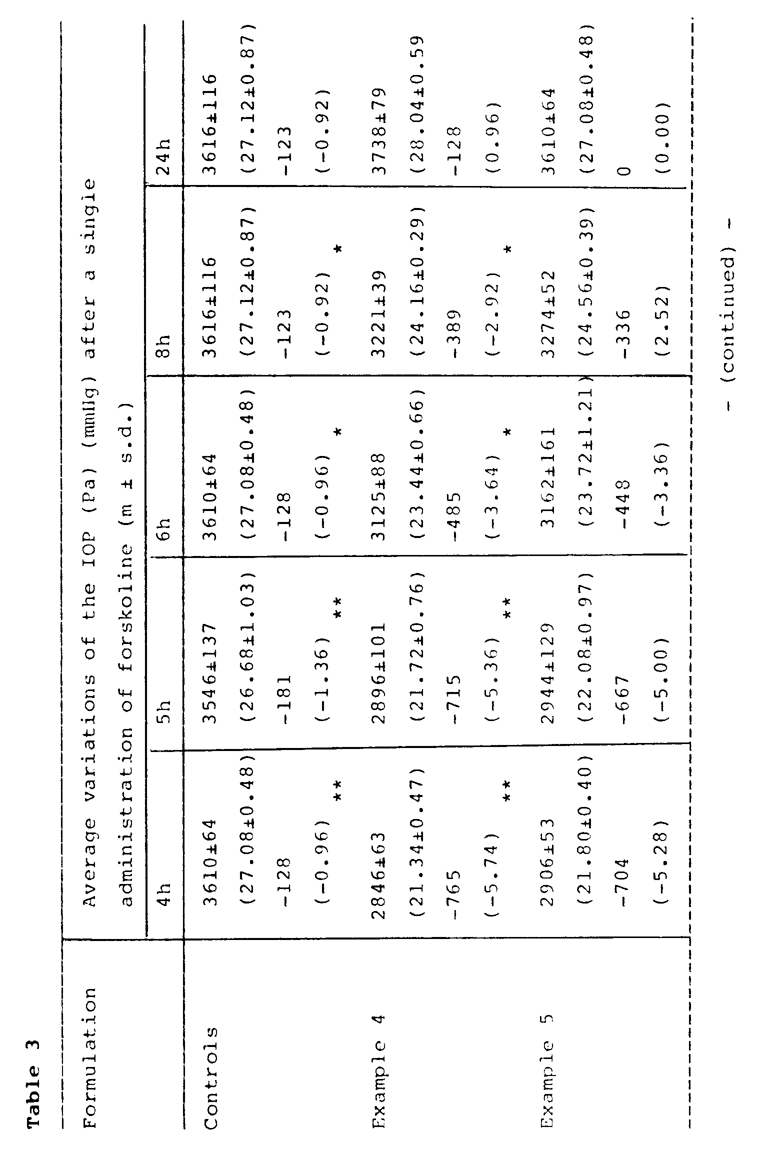

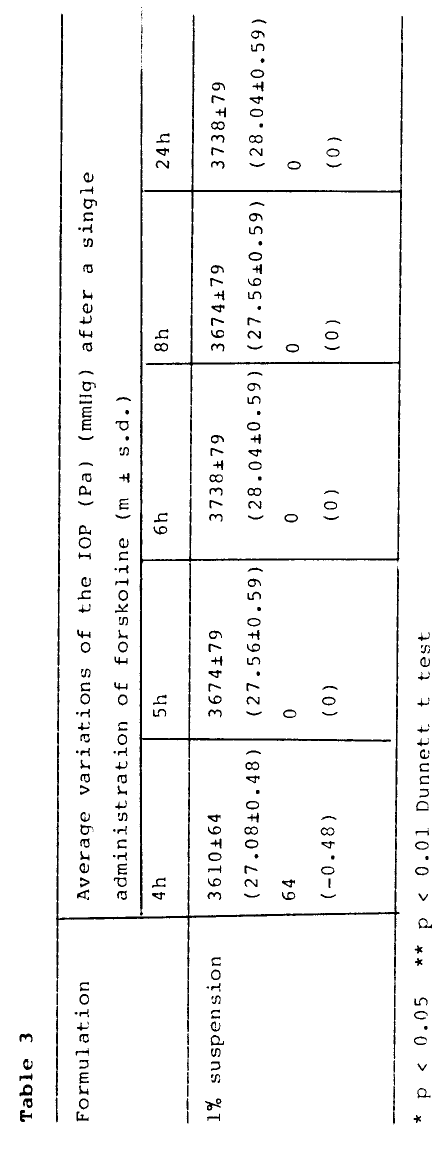

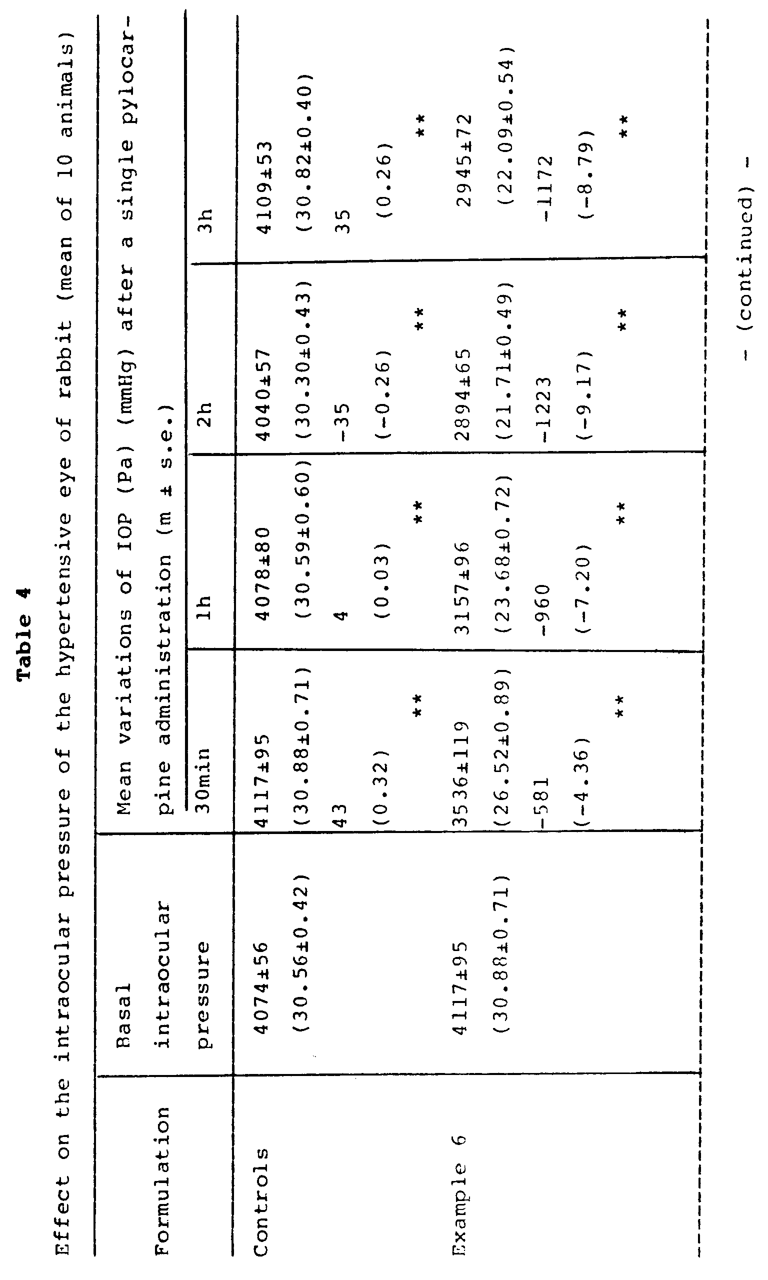

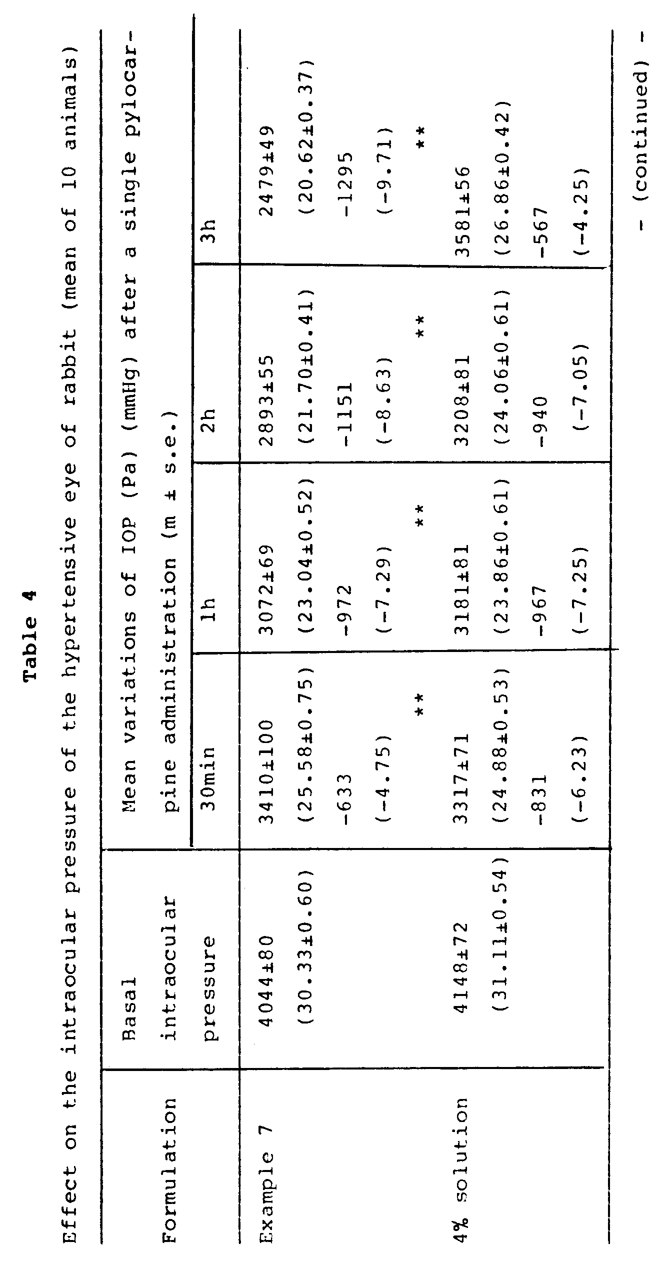

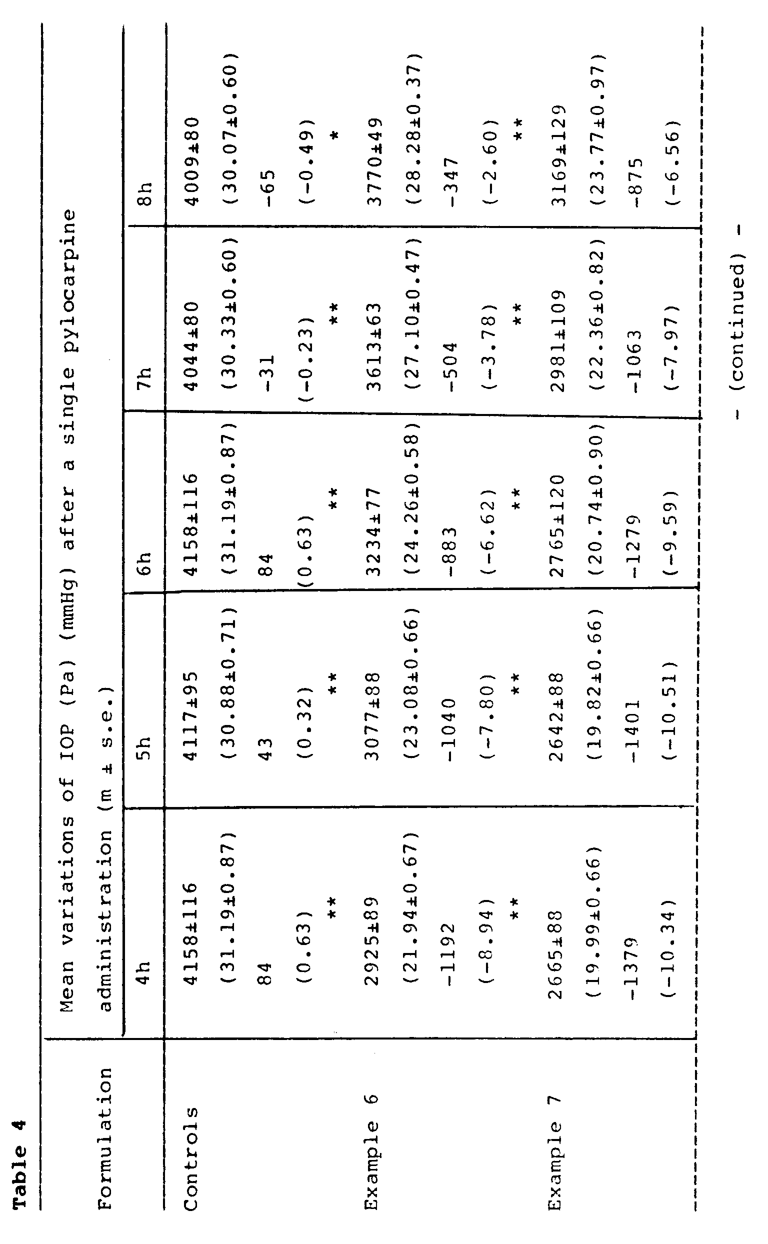

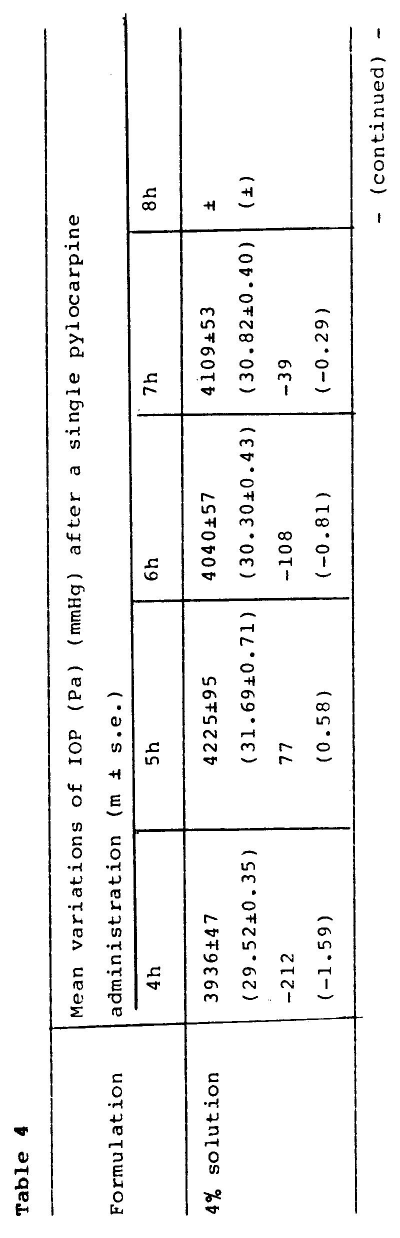

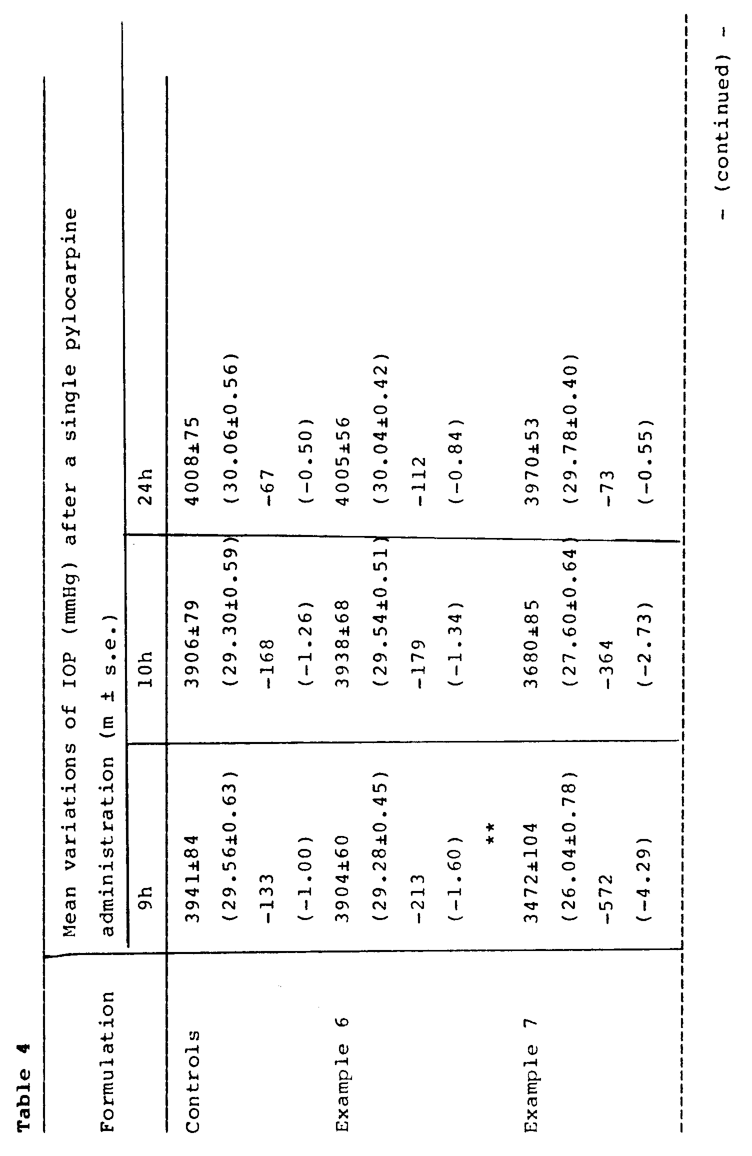

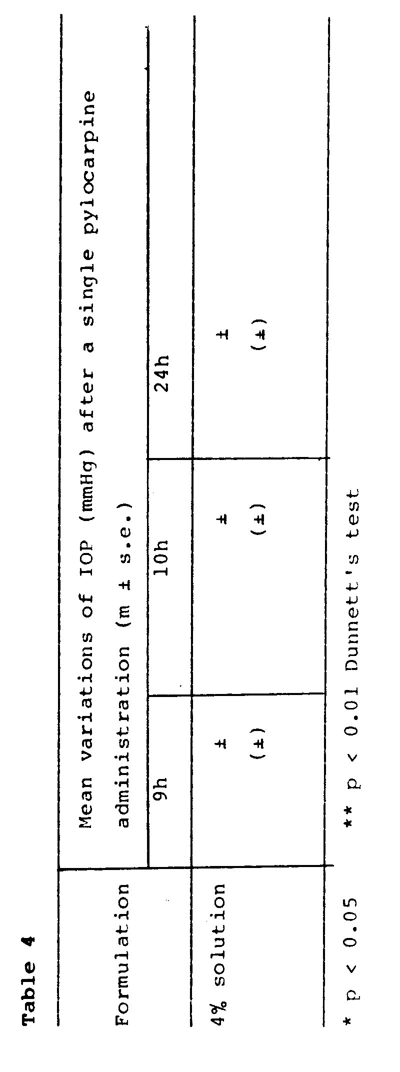

**Table 5**

| Ocular tolerability in the rabbit | | | | | |
|---|---|---|---|---|---|
| Formulation | Average score | | | | |
| | 5 min | 1 h | 4 h | 6 h | 24 h |
| Example 1 | 0 | 2.83 | 1.83 | 1 | 0 |
| Example 4 | 0 | 1.25 | 0.60 | 0.40 | 0 |
| Example 5 | 0 | 1.25 | 0.50 | 0.20 | 0 |
| 0.1% suspension | 2 | 1.00 | 0 | 0 | 0 |
| animals/group: 6 (1) Classification 0 Non-irritant 1 Minimally irritant 2-3 Slightly irritant 4-5 Moderately irritant 6-7 Severely irritant 8-10 Extremely irritant | | | | | |

## Claims

1. Ophthalmic formulations containing active principles in the form of high-viscosity solutions formed of a mixture of water and polyoxyethylene-polyoxyoropylene copolymers (poloxamers) having approximately 70% of polyoxyethylene units and an average molecular weight of approximately 12,500, characterized in that they contain from 5% to 6% (w/v) of non-ionic surfactants as stabilizers.

2. Ophthalmic formulations according to Claim 1 with high therapeutic efficacy and with extended release, containing from 0.01% to 4% w/v of active principle.

3. Ophthalmic formulations according to Claim 1 or 2, characterized in that the non-ionic surfactants are esters of polyoxyethylene sorbitan with fatty acids or polyethoxylated vegetable oils.

4. Ophthalmic formulations according to Claim 3, characterized in that the non-ionic surfactants are selected from polysorbate 20, polysorbate 80 or polyethoxylated hydrogenated castor oil.

5. Ophthalmic formulations according to any one of the preceding claims, in which the poloxamers are present in percentages within the range of 15 to 25% w/v.

6. Ophthalmic formulations according to any one of the preceding claims, characterized in that the polyoxyethylene-polyoxypropylene copolymer is poloxamer 407.

7. Onhthalmic formulations according to any one of the preceding claims, in the form of a gel or of a viscous collyrium.

8. Ophthalmic formulations according to any one of the preceding claims, additionally containing salts, buffering agents and preservatives.

9. Process for the preparation of the ophthalmic formulations of Claims 1-8, which comprises the solubilisation of the active princinle in poloxamers at a temperature of 60-70°C, the addition of the other excipients to the molten mass and cooling to a temperature of 5°C to give a transparent homogeneous liquid.

10. Ophthalmic formulations according to claims 1-8 containing forskoline as active principle for the treatment of glaucoma.

11. Ophthalmic formulations according to claims 1-8 containing thymolol as active principle for the treatment of glaucoma.

12. Ophthalmic formulations according to claims 1-8 containing desamethasone as active principle for the treatment of inflammations of the anterior segment of the eye.

13. Ophthalmic formulations according to claims 1-8 containing flurbiorofen as active principle for the treatment of inflammations of the anterior segment of the eye and in particular for post-operative inflammations.

14. Ophthalmic formulations according to claims 1-8 containing naphazoline as active principle for the treatment of congestive conditions of the conjunctiva.

15. Ophthalmic formulations according to claims 1-8 containing chloroamphenicol as active principle for the treatment of ocular infections.

16. Ophthalmic formulations according to claims 1-8 containing thonzylamine as active principle for the treatment of allergic and inflammatory conditions of the conjunctiva.

17. Ophthalmic formulations according to claims 1-8 containing tetracaine as active principle for the induction of surface anaesthesia.

18. Ophthalmic formulations according to claims 1-8 containing pilocarpine as active principle for the treatment of glaucoma.

## Patentansprüche

1. Ophthalmologische Formulierungen, enthaltend Wirkprinzipien in der Form von hochviskosen Lösungen, die aus einem Gemisch von Wasser und Polyoxyethylen-Polyoxypropylen-Copolymeren (Poloxameren) mit etwa 70% Polyoxyethyleneinheiten und einem mittleren Molekulargewicht von etwa 12.500 gebildet sind, dadurch **gekennzeichnet,** daß sie 5% bis 6% (Gew./Vol.) nichtionische grenzflächenaktive Mittel als Stabilisatoren enthalten.

2. Ophthalmologische Formulierungen nach Anspruch 1, dadurch **gekennzeichnet,** daß sie hohe therapeutische Wirksamkeit und verlängerte Freisetzung aufweisen und 0,01 Gew./Vol.-% bis 4 Gew./Vol.-% Wirkprinzip enthalten.

3. Ophthalmologische Formulierungen nach Anspruch 1 oder 2, dadurch **gekennzeichnet,** daß die nichtionischen grenzflächenaktiven Mittel Ester von Polyoxyethylen-Sorbitan mit Fettsäuren oder polyethoxylierte pflanzliche Öle sind.

4. Ophthalmologische Formulierungen nach Anspruch 3, dadurch **gekennzeichnet,** daß die nichtionischen grenzflächenaktiven Mittel aus Polysorbat 20, Polysorbat 80 oder polyethoxyliertem hydriertem Ricinusöl ausgewählt sind.

5. Ophthalmologische Formulierungen nach einem der vorhergehenden Ansprüche, dadurch **gekennzeichnet,** daß die Poloxamere in Prozentgehalten im Bereich von 15 bis 25 Gew./Vol.-% anwesend sind.

6. Ophthalmologische Formulierungen nach einem der vorhergehenden Ansprüche, dadurch **gekennzeichnet,** daß das Polyoxyethylen-Polyoxypropylen-Copolymere Poloxamer 407 ist.

7. Ophthalmologische Formulierungen nach einem der vorhergehenden Ansprüche, dadurch **gekennzeichnet,** daß sie in der Form eines Gels oder von viskosen Augentropfen vorliegen.

8. Ophthalmologische Formulierungen nach einem der vorhergehenden Ansprüche, dadurch **gekennzeichnet,** daß sie zusätzlich Salze, Puffermittel und Konservierungsmittel enthalten.

9. Verfahren zur Herstellung der ophthalmologischen Formulierungen nach den Ansprüchen 1 bis 8, umfassend die Solubilisierung des Wirkprinzips in Poloxameren bei einer Temperatur von 60 bis 70°C, die Zugabe der anderen Exzipientien zu der geschmolzenen Masse und Kühlen auf eine Temperatur von 5°C zum Erhalt einer transparenten homogenen Flüssigkeit.

10. Ophthalmologische Formulierungen nach den Ansprüchen 1 bis 8, dadurch **gekennzeichnet,** daß sie Forskolin als Wirkprinzip für die Behandlung von Glaukom enthalten.

11. Ophthalmologische Formulierungen nach den Ansprüchen 1 bis 8, dadurch **gekennzeichnet,** daß sie Thymolol als Wirkprinzip für die Behandlung von Glaukom enthalten.

12. Ophthalmologische Formulierungen nach den Ansprüchen 1 bis 8, dadurch **gekennzeichnet,** daß sie Desamethason als Wirkprinzip für die Behandlung von Entzündungen des vorderen Augensegments enthalten.

13. Ophthalmologische Formulierungen nach den Ansprüchen 1 bis 8, dadurch **gekennzeichnet,** daß sie Flurbiprofen als Wirkprinzip für die Behandlung von Entzündungen des vorderen Augensegments und insbesondere von post-operativen Entzündungen enthalten.

14. Ophthalmologische Formulierungen nach den Ansprüchen 1 bis 8, dadurch **gekennzeichnet,** daß sie Naphazolin als Wirkprinzip für die Behandlung von kongestiven Zuständen der Konjunktiva enthalten.

15. Ophthalmologische Formulierungen nach den Ansprüchen 1 bis 8, dadurch **gekennzeichnet,** daß sie Chloramphenicol als Wirkprinzip für die Behandlung von Augeninfektionen enthalten.

16. Ophthalmologische Formulierungen nach den Ansprüchen 1 bis 8, dadurch **gekennzeichnet,** daß sie Thonzylamin als Wirkprinzip für die Behandlung von allergischen und entzündlichen Zuständen der Konjunktiva enthalten.

17. Ophthalmologische Formulierungen nach den Ansprüchen 1 bis 8, dadurch **gekennzeichnet,** daß sie Tetracain als Wirkprinzip für die Einleitung von Oberflächenanästhesie enthalten.

18. Ophthalmologische Formulierungen nach den Ansprüchen 1 bis 8, dadurch **gekennzeichnet,** daß sie Pilocarpin als Wirkprinzip für die Behandlung von Glaukom enthalten.

## Revendications

1. Formulations ophtalmiques contenant des principes actifs sous forme de solutions de haute viscosité formées d'un mélange d'eau et de copolymères de polyoxyéthylène-polyoxypropylène (poloxamères) comprenant environ 70 % de motifs polyoxyéthylène et ayant un poids moléculaire moyen d'environ 12 500, caractérisées en ce qu'elles contiennent de 5 % à 6 % (p/v) d'agents tensio-actifs non ioniques comme stabilisants.

2. Formulations ophtalmiques selon la revendication 1, dotées d'une efficacité thérapeutique élevée et à libération prolongée, contenant de 0,01 % à 4 % p/v de principe actif.

3. Formulations ophtalmiques selon la revendication 1 ou la revendication 2, caractérisées en ce que les agents tensio-actifs sont des esters de polyoxyéthylène sorbitane avec des acides gras ou des huiles végétales polyéthoxylées.

4. Formulations ophtalmiques selon la revendication 3, caractérisées en ce que les agents tensio-actifs non ioniques sont choisis parmi le polysorbate 20, le polysorbate 80 ou l'huile de castor hydrogénée polyéthoxylée.

5. Formulations ophtalmiques selon l'une quelconque des revendications précédentes, dans laquelle les poloxamères sont présents en des quantités comprises entre 15 et 25 % p/v.

6. Formulations ophtalmiques selon l'une quelconque des revendications précédentes, caractérisées en ce que le copolymère polyoxyéthylène-polyoxypropylène est le poloxamère 407.

7. Formulations ophtalmiques selon l'une quelconque des revendications précédentes, sous forme d'un gel ou d'un collyre visqueux.

8. Formulations ophtalmiques selon l'une quelconque des revendications précédentes, contenant en outre des sels, des agents tampons et des conservateurs.

9. Procédé de préparation des formulations ophtalmiques des revendications 1 à 8, qui comprend la solubilisation du principe actif dans des poloxamères à une temperature de 60 à 70°C, l'addition d'autres excipients à la masse fondue et le refroidissement à une température de 5°C pour obtenir un liquide homogène transparent.

10. Formulations ophtalmiques selon les revendications 1 à 8 contenant de la forskoline comme principe actif, pour le traitement du glaucome.

11. Formulations ophtalmiques selon les revendications 1 à 8 contenant du thymolol comme principe actif, pour le traitement du glaucome.

12. Formulations ophtalmiques selon les revendications 1 à 8 contenant de la desaméthasone comme principe actif, pour le traitement d'inflammations du segment antérieur de l'oeil.

13. Formulations ophtalmiques selon les revendications 1 à 8 contenant du flurbiprofène comme principe actif, pour le traitement d'inflammations du segment antérieur de l'oeil et, en particulier, pour les inflammations post-opératoires.

14. Formulations ophtalmiques selon les revendications 1 à 8 contenant de la naphazoline comme principe actif, pour le traitement d'états congestifs de la conjonctive.

15. Formulations ophtalmiques selon les revendications 1 à 8 contenant du chloroamphénicol comme principe actif pour Le traitement d'infections oculaires.

16. Formulations ophtalmiques selon les revendications 1 à 8 contenant de la thonzylamine comme principe actif, pour le traitement d'états allergiques et inflammatoires de la conjonctive.

17. Formulations ophtalmiques selon les revendications 1 à 8 contenant de la tétracaïne comme principe actif, pour l'induction d'anesthésie de surface.

18. Formulations ophtalmiques selon les revendications 1 à 8 contenant de la pilocarpine comme principe actif, pour le traitement du glaucome.
